# EUROPÄISCHE PATENTSCHRIFT

(11) **EP 2 598 468 B1**
(45) Veröffentlichungstag und Bekanntmachung des Hinweises auf die Patenterteilung: **20.01.2016**
(21) Anmeldenummer: 11736403.4
(22) Anmeldetag: 29.07.2011
(51) Int. Cl.: C07C 51/42, C12P 7/56, C12P 7/40

(54) **VERFAHREN ZUR RÜCKGEWINNUNG EINES IN EINEM FERMENTATIONSPROZESS ZUR HERSTELLUNG EINES PRODUKTES EINGESETZTEN HILFSSTOFFS**
METHOD FOR RECOVERING AN AUXILIARY USED IN A FERMENTATION PROCEDURE FOR PRODUCING A PRODUCT
PROCÉDÉ DE RÉCUPÉRATION D'UN ADDITIF UTILISÉ POUR LA FABRICATION D'UN PRODUIT DANS UN PROCESSUS DE FERMENTATION

(30) Priorität: 30.07.2010 EP 10171419
(43) Veröffentlichungstag der Anmeldung: 05.06.2013
(73) Patentinhaber: Pfeifer & Langen Kommanditgesellschaft, 50858 Köln (DE)
(72) Erfinder: KOCH, Timo Johannes, 50189 Elsdorf (DE)
(74) Vertreter: von Kreisler Selting Werner - Partnerschaft von Patentanwälten und Rechtsanwälten mbB
(86) Internationale Anmeldenummer: PCT/EP2011/063131
(87) Internationale Veröffentlichungsnummer: WO 2012/013793

(56) Entgegenhaltungen:
- WO-A1-2010/003534
- DE-A1-102008 032 587
- US-A- 5 641 406
- US-B1- 6 280 985

## Beschreibung

Die vorliegende Erfindung betrifft ein Verfahren zur Rückgewinnung eines in einem Fermentationsprozess zur Herstellung eines Produktes eingesetzten Hilfsstoffs, der einer Fermentationsbrühe zugesetzt worden ist und aus dem mittels einer chemischen Reaktion in der Fermentationsbrühe eine andere Substanz entsteht, wobei die folgenden Schritte durchlaufen werden.

Die fermentative Gewinnung von Ausgangsstoffen zur Herstellung von wertvollen Produkten gewinnt im Zuge der Entwicklung nachhaltiger Verarbeitungsprozesse an Bedeutung. Insbesondere ist die Umsetzung von Saccharose zu organischen Säuren, insbesondere Milchsäure, zu nennen.

Problematisch ist dabei oft nicht nur die Gewinnung der reinen Ausgangsstoffe, sondern die dabei häufig in nicht zu vernachlässigender Menge anfallenden Hilfsstoffe, die teilweise aufwändig entsorgt werden müssen.

Bereits bekannt ist die Aufarbeitung von filtrierten Fermentationsbrühen durch eine Kopplung von monopolarer und bipolarer Elektrodialyse unter Einsatz von Ionentauschern in verschiedenen Stufen des Prozesses, um die Membranverfahren zu schützen. Hier wird das Produkt direkt durch die Membranprozesse von den Hilfsstoffen und Nebenprodukten befreit und das Salz der Milchsäure (oft Natriumlactat) in der bipolaren Elektrodialyse in Milchsäure und Fermentationsbase (oft Natronlauge) gespalten und gleichzeitig beide Stoffe voneinander getrennt. Die Milchsäurelösung wird anschließend durch Eindampfung auf Produktqualität aufkonzentriert. Die Fermentationsbase wird dann ggf. aufkonzentriert und der Fermentation wieder zugeführt. Kennzeichen dieses Prozesses ist ein Recyceln der Fermentationsbase aber die Notwendigkeit den gesamten produkthaltigen Strom über die Membraneinheiten zu leiten (Bailly, M., Production of organic acids by bipolar electrodialysis: realizations and perspectives. Desalination 2002, 144, (1-3), 157-162; Reimann, W., Membrane method for processing fermentation media. F&S, Filtrieren und Separieren 2006, 20, (2), 77-81; Wang, R.; Yu, P.; Li, B.; Wang, H.; Zhang, G.; Guo, T.; Wang, J.; Zhang, J. The sodium salt method and electrodialysis for extracting lactic acid from fermentation broth. 2008-10049647; CN: 101294169, 20080428., 2008).

Ebenfalls bekannt ist der Einsatz einer Simulated Moving Bed (SMB) Chromatographie in der Aufarbeitung von Fermentationsbrühen aus der Milchsäureherstellung. Hier wird die gefilterte Fermentationsbrühe zunächst mit Mineralsäure (H₂SO₄) angesäuert, um aus dem Salz der Milchsäure (Ammoniumlactat) die freie Säure zu erhalten. Diese Lösung wird dann durch die SMB Chromatographie in einen Milchsäurestrom und einen Salzstrom mit Nebenprodukten aufgetrennt. Der Milchsäurestrom wird anschließend ggf. weiter aufgereinigt und durch Eindampfung auf Produktkonzentration eingeengt. Der Nebenproduktstrom wird auskristallisiert und fällt somit als Koppelprodukt an. Der Prozess ist wesentlich durch eine robuste und nahezu verlustfreie Trennung der Milchsäure von den Nebenprodukten gekennzeichnet, wobei nachteiligerweise allerdings zwangsweise Koppelprodukt in vergleichbarer Quantität wie das Hauptprodukt anfällt (Tietz, W.; Schulze, J.; Klecha, R. Purification of lactic acid using simulated moving bed ion exchange chromatography, 2010; (DE 102008032587). WO-A-2010-003534 offenbart ein Verfahren zur Aufarbeitung organischer Säuren aus Fermentationsbrühen, wobei der pH-Wert während der Fermentation über die Zugabe einer Base eingestellt wird, welche die entstehende organische Säure als Salz bindet, die Biomasse von der Fermentationsbrühe abgetrennt wird und das entstehende Filtrat mit Säure behandelt wird, wodurch ein Gemisch entsteht, das die freigesetzte organische Säure sowie weitere Komponenten enthält. Das Gemisch wird auf einen mit einer geeigneten mobilen Phase äquilibriertes Chromatographieharz, eine kontinuierlich betriebene Simulated Moving Bit-Chromatographie, umfassend eine oder mehrere miteinander verbundene Säulen aufgebracht und die Säule mit der mobilen Phase gespült. Es werden verschiedene Fraktionen erhalten, in denen Einzelkomponenten des Gemisches aufgrund unterschiedlicher Retentionszeiten getrennt voneinander gesammelt werden. Die US-A-5,641,406 offenbart ein Verfahren zur Abtrennung und Reinigung von Milchsäure aus salz- und kohlenhydrathaltigen, von grobdispersen und lipophilen Verunreinigungen befreiten Fermentationslösungen, wobei der Abtrennungs- und Reinigungsprozess in zwei Schritten vorgenommen wird und wobei im ersten Schritt, in einen oder mehreren Vorsäulen, die in der Fermentationslösung gegebenenfalls vorhandenen Salze, vor allem das Salz der Milchsäure, durch einen Ionenaustauscherschritt in die freien Säuren überführt und im zweiten Schritt, in einer oder mehreren Trennsäulen, die freie Milchsäure durch Chromatographie an stark saurem Ionenaustauscher von den übrigen in der Fermentationslösung vorhandenen Säuren, Kohlenhydraten und sonstigen Verunreinigungen getrennt wird. Zwar erwähnt die genannte Druckschrift, dass eine salzspaltende Elektrodialyse verwendet werden kann, rät aber ausdrücklich von der Verwendung der Elektrodialyse ab, da diese nachteilig ist aufgrund der Benötigung großer Mengen elektrischer Energie und zusätzliche Reinigungsschritte verlangt (Spalte 4, Zeile 54-58).

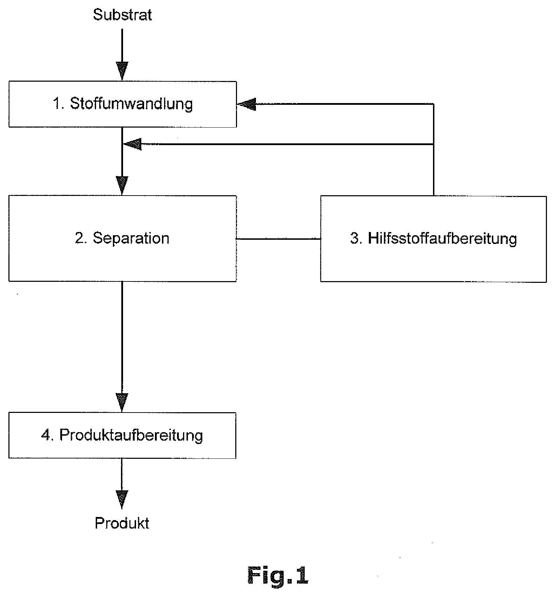

Das der Erfindung zugrunde liegende Problem besteht darin, ein Verfahren anzugeben, das es erlaubt Fermentationsbrühen, insbesondere aus der Milchsäurevergärung nach Abtrennung von Mikroorganismen soweit aufzuarbeiten, dass das Fermentationsprodukt, insbesondere die Milchsäure in einer Qualität bereitgestellt werden kann, wie sie für die Weiterverarbeitung des Fermentationsprodukts, insbesondere die Polymerisation von Milchsäure zu Polymilchsäure (PLA) benötigt wird. Darüber hinaus soll der Prozess so gestaltet sein, dass möglichst alle wesentlichen Hilfsstoffe in Kreisläufen gefahren und somit regeneriert werden können (Fig. 1).
Figur 1 zeigt in allgemeiner Form einen erfindungsgemäßen Verfahrensablauf.
Figur 2 zeigt eine schematische Darstellung des erfindungsgemäßen Verfahrens in Einzelheiten.

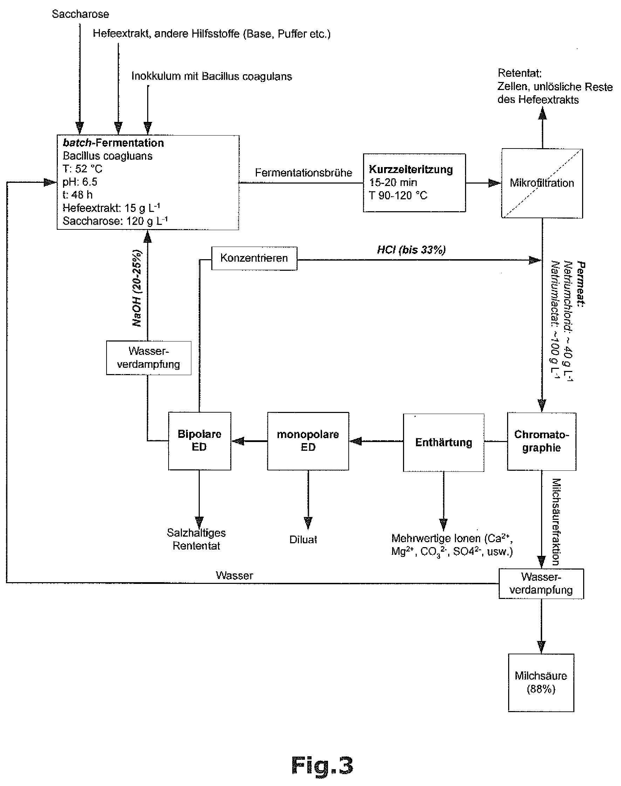

Figur 3 zeigt ein Ausführungsbeispiel des erfindungsgemäßen Verfahrens betreffend die Herstellung von Milchsäure nach dem erfindungsgemäßen Verfahren.
Figur 4 zeigt ein Konzentrationsprofil der Chromatographie.

Gegenstand der vorliegenden Erfindung ist ein Verfahren zur Rückgewinnung eines in einem Fermentationsprozess zur Herstellung eines Produktes eingesetzten Hilfsstoffs, der einer Fermentationsbrühe zugesetzt worden ist und aus dem mittels einer chemischen Reaktion in der Fermentationsbrühe eine andere Substanz entsteht, wobei die folgenden Schritte durchlaufen werden:
- die Fermentationsbrühe wird einem ersten Verfahrensschritt unterzogen, in dem der in dem Fermentationsprozess eingesetzte Hilfsstoff, ein saurer Ionenaustauscher, eine Minerallauge oder Mineralsäure ist, ausgewählt aus der Gruppe bestehend aus Natronlauge, Ammoniak, Kalilauge, Salzsäure, Schwefelsäure, oder Phosphorsäure, und die entstehende andere Substanz ausgewählt ist aus der Gruppe bestehend aus Natrium-, Kalium,- Ammoniumchlorid, Natrium-, Kalium, Ammoniumphosphat, Natrium-, Kalium,- Ammoniumsulfat und/oder zumindest teilweise erschöpften sauren Ionentauschern mittels mindestens einem chromatographischen Verfahren in eine Fraktion A von dem mindestens einen in dem Fermentationsprozess anfallenden Produkt getrennt wird und letzteres in einer Fraktion B anfällt,
- die Fraktion A in einem zweiten Verfahrensschritt einer bipolaren Elektrodialyse unterworfen wird, um aus in der Fraktion A enthaltenen aus dem Hilfsstoff entstandene andere Substanz, wie Salzen, den Hilfsstoff, wie die zu den Salzen korrespondierenden Säuren/Laugen, zu generieren.

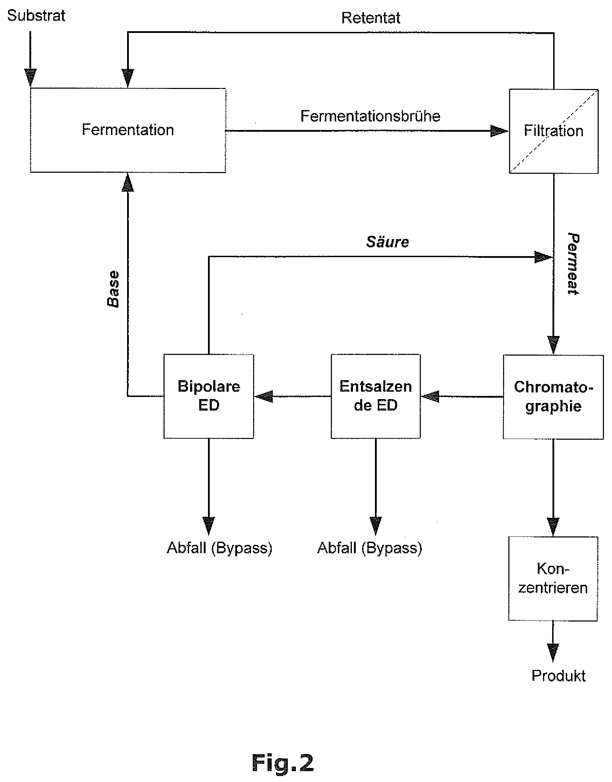

Dies wird in Figur 2 durch das wiedergegebene Fließschema illustriert.

In einer Ausführungsform des erfindungsgemäßen Verfahrens werden in einem dem ersten Verfahrensschritt vorgelagerten Schritt nicht in der Fermentationsbrühe gelöste Bestandteile in eine Fraktion C abgetrennt. Die Abtrennung erfolgt beispielsweise durch ein mechanisches Trennverfahren, wie Filtration. Es werden dadurch Mikroorganismen und weitere unlösliche Bestandteile, wie anorganische Verunreinigungen aus Substrat und/oder Nährstoffquelle, wozu Sand und andere Erdbestandteile oder auch Zellmaterial von Pflanzen und/ oder Mikroorganismen und/oder Enzyme zählen können, abgetrennt. Diese Fraktion kann in einem kontinuierlichen Verfahren dem Fermenter wieder zugeführt werden, wodurch die Mikroorganismen erneut beispielsweise zur weiteren Synthese von Produkten, wie Milchsäure, Bernsteinsäure, Itaconsäure, Zitronensäure und anderen organischen Säuren eingesetzt werden können. In diskontinuierlichen Verfahren kann die Fraktion C weiter getrocknet und entweder z. B. thermisch verwertet oder als Futtermittel eingesetzt werden.

In einer anderen Ausführungsform des erfindungsgemäßen Verfahrens wird zur Abtrennung von nicht in der Fermentationsbrühe gelösten Bestandteilen mindestens eine Filtration oder Ultrafiltration eingesetzt.

In einer weiteren Ausführungsform des erfindungsgemäßen Verfahrens sind in der Fraktion C Zellen, Enzyme, unlösliche Stärke, Nährstoffe (Pepton, Peptide aus Fleischextrakt oder Hefezellen) vorhanden.

Typischerweise können im erfindungsgemäßen Verfahren die Hilfsstoffe wieder in den Fermentationsprozess eingeschleust werden, insbesondere nach Einstellung einer vorherbestimmten Konzentration. Die einzustellenden Konzentrationen hängen vom Fermentationstyp oder verfahrenstechnischen Randbedingungen ab, die dem Fachmann bekannt sind, sodass dem Fachmann bewusst ist, welchen Wert die Konzentration der Hilfsstoffe annehmen soll. Die Konzentration liegt für die Fermentationsbase und die vor dem ersten Verfahrensschritt zugegebene Säure bei 15-40%.

Erfindungsgemäß ist, dass mindestens ein bei der Fermentation anfallendes Produkt, insbesondere Milchsäure, Bernsteinsäure, Itaconsäure, Zitronensäure oder eine andere organische Säure später isoliert wird. Die Produkte werden durch Verstoffwechselung des Substrats, einer Kohlenhydratquelle, wie Cellulose, Stärke, Stärkehydrolysate, Glukose/- Fruktosesirupe oder Saccharose in Form von reiner Lösung oder Rohsaft, Dünnsaft oder Rübendicksaft, durch insbesondere spezialisierte Mikroorganismen, wie Lactobacillen insbesondere Lactococcen produziert. Üblicherweise liegt die Produktkonzentration nach der Fermentation zwischen 10% und 30%.
Die Abtrennung des Produkts in eine Fraktion B erfolgt zweckmäßigerweise nach dem ersten Verfahrensschritt. Die Konzentration des Produkts liegt z. B. zwischen 10% und 30%. Die Fraktion B kann in einer Verfahrensvariante durch insbesondere mehrstufiges Verdampfen z. B. bei erhöhter Temperatur und/oder unter vermindertem Druck auf eine Produktkonzentration von 70%-90% konzentriert werden.

Die aus einem in dem Fermentationsprozess eingesetzten Hilfsstoff entstehende andere Substanz ist Natrium-, Kalium, Ammoniumchlorid, NatriumKalium-, Ammoniumphosphat und/oder Natrium-, Kalium-, Ammoniumsulfat. Sie entsteht durch Ansäuern des Produktsalzes mit einer Säure, wobei das Produkt zur freien organischen Säure protoniert wird und das Anion der Säure mit dem Kation des Produktsalzes die entsprechenden zuvor genannten Substanzen bildet. Der pH-Wert liegt nach dem Ansäuern üblicherweise zwischen pH:0 und pH:4; (0≤pH≤4).

Als Hilfsstoff werden im erfindungsgemäßen Verfahren Minerallaugen und Mineralsäuren, wie Natronlauge, Ammoniak, Kalilauge, Salzsäure, Schwefelsäure, Phosphorsäure, eingesetzt. Mit den Säuren kann die Fermentationsbrühe nach Abtrennung der unlöslichen Bestandteile in die Fraktion C von pH:5 bis pH:7 auf pH:0 bis pH:4 angesäuert werden.

Die im erfindungsgemäßen Verfahren als Hilfsstoff neben Minerallaugen oder Mineralsäuren einsetzbaren Ionenaustauscher gehören zum Typ der sauren Ionenaustauscher. Zu nennen sind dabei insbesondere handelsübliche Produkte wie Amberlite IR100, 120, Amberjet 1000, 1200, Dowex HCR-W2, die in an sich bekannter Weise regeneriert werden können.

In einer weiteren Ausführungsform des erfindungsgemäßen Verfahrens ist das mindestens eine chromatographische Verfahren eine SMB-Chromatographie, oder Chromatographie mit Ionentauschern. Als Trennphase werden insbesondere ionische Harzpolymere von Styrol-Divinylbenzolcopolymere, wie Amberlite FPA53 oder Amberlite CR 5550 eingesetzt. Dieser Verfahrensschritt kann auch mit anorganischen Materialien, wie SiO₂ oder Al₂O₃ als Trennphase durchgeführt werden.

In noch einer Ausführungsform des erfindungsgemäßen Verfahrens wird zwischen dem ersten und dem zweiten Verfahrensschritt ein Enthärtungsschritt, insbesondere Enthärtung mit einem Ionentauscher, wie S950 der Firma Purolite, zur Entfernung von Prozesschemikalien eingesetzt. In diesem Schritt können mehrwertige Ionen, wie Mg²⁺, Ca²⁺ etc. aus der Fraktion A abgetrennt werden. In der Fraktion A verbleiben üblicherweise Salz, das aus Fermentationsbase und zugegebener Säure gebildete Salz, insbesondere Natrium-, Kalium, Ammoniumchlorid, Natrium- Kalium-, Ammoniumphosphat und/oder Natrium-, Kalium-, Ammoniumsulfat, sowie nichtionische Bestandteile aus der Fermentation, die nicht über die Chromatographie abgetrennt werden.

In noch einer Ausführungsform des erfindungsgemäßen Verfahrens wird zwischen dem ersten und zweiten Verfahrensschritt ein Entsalzungsschritt, insbesondere eine monopolare Elektrodialyse zur Aufkonzentrierung und Abtrennung von Prozesschemikalien, insbesondere nichtionischere Bestandteile eingesetzt. In diesem Schritt können nichtionische Verbindungen, wie nicht umgesetztes Substrat, Fermentationshilfsstoffe (Nährstoffe, Proteine etc.) abgetrennt werden und das in der Fraktion A üblicherweise verbleibende Salz, das aus Fermentationsbase und zugegebener Säure gebildete Salz, insbesondere Natrium-, Kalium, Ammoniumchlorid, Natrium- Kalium-, Ammoniumphosphat und/oder Natrium-, Kalium-, Ammoniumsulfat aufkonzentriert werden.

### Beispiel

Die Erfindung wird an Hand der Milchsäurefermentation näher beschrieben . Eine typische Fermentation, die üblicherweise unter erheblichem Verbrauch von Hilfsstoffen und Anfall von Koppelprodukt arbeitet, ist die Herstellung von Milchsäure.

In einem 7 L Fermenter, Sartorius Biostat B Plus wird 120 g L⁻¹ Kristallzucker mit 15 g L⁻¹ Hefeextrakt (Deutsche Hefewerke GmbH/Germany, No. 20901001) mit *Bacillus coagulans* zu Milchsäure fermentiert. Das Inokulum beträgt 2% des Fermentervolumens und wird durch Anzucht von *Bacillus coagulans* in 20 g L⁻¹ Saccharoselösung mit 15 g L⁻¹ Hefeextrakt hergestellt. Die Fermentation wird bei 52,0 °C, pH 6,5 und 200 rpm über 27 h durchgeführt. Der pH Wert wird durch Zugabe 25%iger Natronlauge konstant gehalten. Nach Ablauf der 27 h Reaktionszeit wird die Fermentationsbrühe über einen Kurzzeiterhitzer für 15 min auf 90 °C erwärmt, um die Mikroorganismen zu inaktivieren. Die Fermentationsbrühe wird dann über einem Mikrofiltrationsmodul (UMP-1047R, Firma Pall) filtriert und unlösliche Bestandteile, wie die Mikroorganismen dabei abgetrennt. Die Milchsäurekonzentration im Filtrat wird per HPLC mit 102,4 g L⁻¹ bestimmt. Das Filtrat wird anschließend mit 33%iger Salzsäure auf pH 3,7 angesäuert und zur Chromatographie gegeben. Nach HPLC Analyse liegt die Milchsäurekonzentration hier bei 97,7 g L⁻¹, die Saccharose ist per HPLC nicht mehr nachweisebar. In Tab. 1 ist die Zusammensetzung im Filtrat gelösten Ionen gezeigt, die durch Ionenchromatographie bestimmt wurden.

**Tau.1: Anionen und Kationen im angesäuerten Filtrat der Fermentationsbrühe**

| Bezeichnung | Konzentration |
|---|---|
| Natrium | 22,732 g L⁻¹ |
| Kalium | 0,354 g L⁻¹ |
| Magnesium | 0,018 g L⁻¹ |
| Calcium | 0,024 g L⁻¹ |
| Ammonium | 0,059 g L⁻¹ |
| Chlorid | 19,415 g L⁻¹ |
| Phosphat | 0,012 g L⁻¹ |
| Sulfat | 0,079 g L⁻¹ |
| Nitrit | 0,034 g L⁻¹ |
| Nitrat | <0,001 g L⁻¹ |

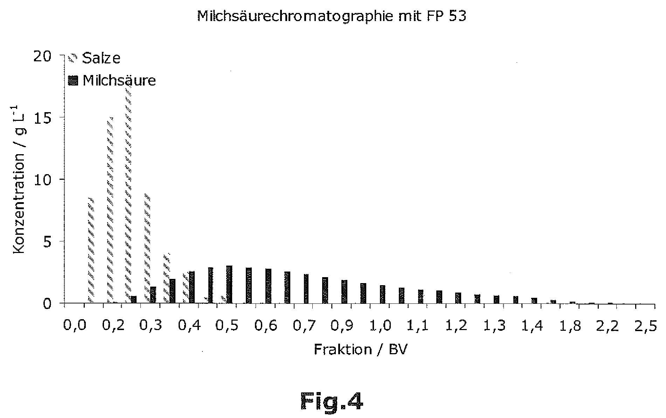

Die angesäuerte Fermentationsbrühe wird nun zur vollständigen Protonierung der Milchsäure weiter mit 33%iger Salzsäure auf pH 2,07 angesäuert. Die so vorbereitete Lösung wird dann über eine Chromatographiesäule mit Bettvolumen (BV) von 0,85 L chromatographiert. Als Trennharz wird Amberlite FP53 (Firma Rohm und Haas) oder Amberlite CR5550 verwendet. Die Beladung beträgt 0,03 BV. Als Eluens wird destilliertes Wasser oder eine 0,02N H₂SO₄-Lösung eingesetzt. Nach einem Vorlauf mit einer Leitfähigkeit <100µS cm⁻¹ werden aufgetrennten Komponenten der Ausgangsmischung in Fraktionen von jeweils 50 mL aufgefangen. Das Konzentrationsprofil der Chromatographie ist in Figur 4 gezeigt. In den ablaufenden Fraktionen werden zunächst die Salze, danach die milchsäurehaltige Fraktion erhalten, die in weiteren Stufen auf die erforderliche Produktkonzentration von 88% durch Wasserverdampfung aufkonzentriert wird und ggf. weiter durch weitere Prozessstufen noch gereinigt werden kann. Nach Abtrennung der Milchsäurefraktion wird das Harz mit 4%iger NaOH Lösung regeneriert. Das natriumchloridhaltige Regenerat wird zur Salzfraktion hinzugegeben.Die Salzfraktion wird gesammelt und den weiteren Schritten zur Rückgewinnung der Hilfsstoffe, Natronlauge und Salzsäure zugeführt.

Nach der Chromatographie wird über die Schritte Enthärtung, monopolare und bipolare Elektrodialyse die Lösung um den Faktor 1,67 mit Wasser verdünnt. Nach der bipolaren Elektrodialyse werden drei Fraktionen, eine Säure, eine Lauge und eine saure salzhaltige Fraktion, als Retentat gewonnen. Das Verhältnis dieser Fraktionen ist: 1,3 Volumenanteile Säure auf 1 Volumenanteil Lauge und 0,3 Volumenanteile Salzfraktion auf 1 Volumenanteil Säure. Der Gehalt an Saccharose, Glukose, Fruktose oder Milchsäure liegen in diesen Fraktionen unterhalb der Nachweisegrenze der HPLC.

Die salzhaltigen Fraktionen aus der Chromatographie, die im weiteren Verlauf insbesondere in eine Säure und eine Laugefraktion aufgetrennt werden, werden zunächst zum Schutz der nachfolgenden Prozessstufen enthärtet. Zur Enthärtung werden 2 L Ionentauscherharz (S950, Firma Purolite) eingesetzt und mit 5 BV h⁻¹ senkrecht von unten nach oben durchströmt.

Die enthärtete Lösung wird anschließend als Diluatstrom einer monopolaren Elektrodialyseeinheit (Firma Deukum, Typ 100) zugeführt. Die Anlage enthält je 10 Anionen- und Kationentauschermembranen der Firma Fumatec (Typ100 quadro) mit einer effektiven Gesamtfläche von 0,21 m².

Der Konzentratbehälter ist zunächst mit VE-Wasser gefüllt, das durch Zugabe von HCl auf eine Leitfähigkeit von 1,0 mS cm⁻¹ eingestellt wird. Die Spülung der Elektroden erfolgt mit einer Natriumsulfatlösung (Leitfähigkeit 10 mS cm⁻¹). Durch Anlegen der Spannung wandern die im Diluat gelösten Ionen durch die Membranen in den Konzentratbehälter. Die Grenzwerte für Spannung und Strom liegen bei 20 V und 3,0 A. Die Trennung wird nach Unterschreiten einer Leitfähigkeit von 3 mS cm⁻¹ im Diluat beendet.

Durch die monopolare Elktrodialyse wird die salzhaltige Lösung aufkonzentriert. Neben dem Konzentrat bleibt noch das Diluat zurück, das noch in geringen Konzentrationen Ionen enthält. Die Zusammensetzung des Konzentrats, das anschließend in der bipolaren Elektrodialyse weiter verarbeitet wird ist in Tab. 2 angegeben. Durch die monopolare Elektrodialyse werden die Ionen so aufkonzentriert, dass ca. 73% des Wassers entfernt werden.

**Tab. 2: Anionen und Kationen im Konzentrat der monopolaren Elektrodialyse**

| Bezeichnung | Konzentration |
|---|---|
| Natrium | 48,162 g L⁻¹ |
| Kalium | 2,800 g L⁻¹ |
| Magnesium | <0,001 g L⁻¹ |
| Calcium | 0,003 g L⁻¹ |
| Ammonium | 0,494 g L⁻¹ |
| Phosphat | 0,488 g L⁻¹ |
| Chlorid | 64,012 g L⁻¹ |
| Sulfat | 0,189 g L⁻¹ |
| Nitrit | n.b.* |
| Nitrat | 0,002 g L⁻¹ |

| | |
|---|---|
| *n.b.: nicht bestimmt | |

Die Zusammensetzung des Diluats ist in Tab. 3 angegeben.

**Tab. 3: Anionen und Kationen im Diluat der monopolaren Elektrodialyse**

| Bezeichnung | Konzentration |
|---|---|
| Natrium | 0,853 g L⁻¹ |
| Kalium | 0,030 g L⁻¹ |
| Magnesium | <0,001 g L⁻¹ |
| Calcium | <0,001 g L⁻¹ |
| Ammonium | 0,018 g L⁻¹ |
| Phosphat | 0,231 g L⁻¹ |
| Chlorid | 0,743 g L⁻¹ |
| Sulfat | 0,042 g L⁻¹ |
| Nitrit | 0,004 g L⁻¹ |
| Nitrat | <0,001 g L⁻¹ |

Die bipolare Elektrodialyse wird ebenfalls auf einer Anlage der Firma Deukum durchgeführt. Die Konzentratfraktion aus der monopolaren Elektrodialyse wird in den Salzkreislauf der bipolaren Elektrodialyse vorgelegt. Der Säure und Laugekreislauf wird jeweils mit VE Wasser befüllt und durch Zugabe von HCl zu ersterem eine Leitfähigkeit von 1 mS cm⁻¹, bzw. durch Zugabe von NaOH zu letzterem eine Leitfähigkeit von 50 mS cm⁻¹ eingestellt. Die Spülung der Elektroden wird mit einer verdünnten NaOH Lösung (Leitfähigkeit 50 mS cm⁻¹) durchgeführt. Nach Anlegen der Spannung wandern die Anionen in den Säurekreislauf und die Kationen in den Laufekreislauf. Die Spannungs- und Stromgrenzen liegen hier bei 30 V, bzw. 5 A. Die Apparatur arbeitet mit jeweils 10 Kationen-, Anionen- und bipolaren Membranen der Firma Fumatec (Typ 100).

Nach Erreichen einer Leitfähigkeit von 250 mS cm⁻¹ im Säurekreislauf, bzw. 220 mS cm⁻¹ im Laugekreislauf wird ein Teil der entstandenen Säure und Lauge entnommen und durch VE Wasser ersetzt, um das Konzentrationsgefälle der Ionen über die Membrane wieder anzuheben und den Ionentransport damit zu beschleunigen. Die bipolare Elektrodialyse wird beendet, nachdem die Leitfähigkeit im Salzstrom unter 25 mS cm⁻¹ gesunken ist.

Aus der bipolaren Elektrodialyse wird eine 5,2%ige Natronlauge gewonnen. Der pH Wert liegt bei 13,22. Die Lauge enthält noch folgende Ionen (Tab. 4):

**Tab. 4: Anionen und Kationen in der durch bipolare Elektrodialyse gewonnenen Natronlauge (ohne Na⁺)**

| Bezeichnung | Konzentration |
|---|---|
| Kalium | 1,636 g L⁻¹ |
| Magnesium | <0,001 g L⁻¹ |
| Calcium | 0,010 g L⁻¹ |
| Ammonium | 0,151 g L⁻¹ |
| Chlorid | 0,441 g L⁻¹ |
| Phosphat | <0,001 g L⁻¹ |
| Sulfat | <0,001 g L⁻¹ |
| Nitrit | <0,001 g L⁻¹ |
| Nitrat | <0,001 g L⁻¹ |

Durch Wasserverdampfung wird die 5,2 %ige Natronlauge auf 21,2% NaOH konzentriert. Die Verdampfung wird in einer Vakuumdestillationsanlage der Firma Büchi, bei 55 °C, 50 mbar und 200 rpm durchgeführt.
Als zweite Fraktion wird aus der bipolaren Elektrodialyse eine 6,78%ige Salzsäurelösung mit pH<1 gewonnen. Die Anteile der weiteren ionischen Bestandteile sind in Tab. 5 aufgelistet.

**Tab. 5: Anionen und Kationen in der durch bipolare Elektrodialyse gewonnenen Salzsäure (ohne Cl⁻)**

| Bezeichnung | Konzentration |
|---|---|
| Natrium | 0,767 g L⁻¹ |
| Kalium | 0,064 g L⁻¹ |
| Magnesium | <0,001 g L⁻¹ |
| Calcium | 0,005 g L⁻¹ |
| Ammonium | 0,038 g L⁻¹ |
| Phosphat | 0,037 g L⁻¹ |
| Sulfat | 0,061 g L⁻¹ |
| Nitrit | 0,033 g L⁻¹ |
| Nitrat | <0,001 g L⁻¹ |

Als Retentat der bipolaren Elektrodialyse bleibt eine salzhaltige saure Fraktion mit pH 1,48. Die Konzentrationen der im Retentat gelösten Ionen sind in Tab.6 aufgeführt.

**Tab. 6: Anionen und Kationen aus dem Retentat der bipolaren Elektrodialyse**

| Bezeichnung | Konzentration |
|---|---|
| Natrium | 2,981 g L⁻¹ |
| Kalium | 0,038 g L⁻¹ |
| Magnesium | <0,001 g L⁻¹ |
| Calcium | 0,002 g L⁻¹ |
| Ammonium | 0,007 g L⁻¹ |
| Phosphat | 0,765 g L⁻¹ |
| Chlorid | 6,583 g L⁻¹ |
| Sulfat | 0,078 g L⁻¹ |
| Nitrit | 0,029 g L⁻¹ |
| Nitrat | 0,001 g L⁻¹ |

Im nächsten Fermentationszyklus wird der Fermenter wieder mit einer Brühe aus 120 g L⁻¹ Saccharose als Substrat und 15 g L⁻¹ Hefeextrakt als Nährstoffquelle befüllt. Angeimpft wird mit einem Inokkulum von 2% des Fermentervolumens (*Bacillus coagulans* in 20 g L⁻¹ Saccharose und 15 g L⁻¹ Hefeextrakt). Zur pH-Korrektur wird die zuvor zurückgewonnene 21,2%ige Natronlauge eingesetzt. Nach 30 h kann per HPLC keine Saccharose mehr in der Fermentationsbrühe nachgewiesen werden. Die Mikroorganismen werden durch Kurzzeiterhitzung bei T>90 °C für mind. 15 min inaktiviert und die Zellen über die beschriebene Mikrofiltration abgetrennt. Die Milchsäurekonzentration wird hieraus per HPLC mit 96,1 g L⁻¹ bestimmt.

## Patentansprüche

1. Verfahren zur Rückgewinnung eines in einem Fermentationsprozess zur Herstellung eines Produktes eingesetzten Hilfsstoffs, der einer Fermentationsbrühe zugesetzt worden ist und aus dem mittels einer chemischen Reaktion in der Fermentationsbrühe eine andere Substanz entsteht, wobei die folgenden Schritte durchlaufen werden:
• die Fermentationsbrühe wird einem ersten Verfahrensschritt unterzogen, in dem der in dem Fermentationsprozess eingesetzte Hilfsstoff, ein saurer Ionenaustauscher, eine Minerallauge oder Mineralsäure ist, ausgewählt aus der Gruppe bestehend aus Natronlauge, Ammoniak, Kalilauge, Salzsäure, Schwefelsäure, oder Phosphorsäure, und die entstehende andere Substanz ausgewählt ist aus der Gruppe bestehend aus Natrium-, Kalium,- Ammoniumchlorid, Natrium-, Kalium, Ammoniumphosphat, Natrium-, Kalium,- Ammoniumsulfat und/oder zumindest teilweise erschöpften sauren Ionentauschern mittels mindestens einem chromatographischen Verfahren in eine Fraktion A von dem mindestens einen in dem Fermentationsprozess anfallenden Produkt getrennt wird und letzteres in einer Fraktion B anfällt,
• die Fraktion A in einem zweiten Verfahrensschritt einer bipolaren Elektrodialyse unterworfen wird, um aus in der Fraktion A enthaltenen aus dem Hilfsstoff entstandene andere Substanz, wie Salzen, den Hilfsstoff, wie die zu den Salzen korrespondierenden Säuren/Laugen, zu generieren.

2. Verfahren nach Anspruch 1, wobei in einem dem ersten Verfahrensschritt vorgelagerten Schritt nicht in der Fermentationsbrühe gelöste Bestandteile in eine Fraktion C abgetrennt werden.

3. Verfahren nach Anspruch 2, wobei zur Abtrennung von nicht in der Fermentationsbrühe gelösten Bestandteilen mindestens eine Filtration oder Ultrafiltration eingesetzt wird.

4. Verfahren nach mindestens einem der Ansprüche 1 bis 3, wobei in der Fraktion C Zellen, Enzyme, unlösliche Stärke, Nährstoffe wie Pepton, Peptide aus Fleischextrakt oder Hefezellen oder Hefehydrolysat oder Hefeextrakt vorhanden sind.

5. Verfahren nach mindestens einem der Ansprüche 1 bis 4, wobei die Hilfsstoffe wieder in die Fermentation eingeschleust werden, insbesondere nach Einstellung einer vorherbestimmten Konzentration.

6. Verfahren nach mindestens einem der Ansprüche 1 bis 5, wobei das mindestens eine in dem Fermentationsprozess anfallende Produkt, Milchsäure, Bernsteinsäure, Itakonsäure, Zitronensäure und andere organische Säuren ist.

7. Verfahren nach mindestens einem der Ansprüche 1 bis 6, wobei der Hilfsstoff Natronlauge, Ammoniak, Kalilauge, Salzsäure, Schwefelsäure, Phosphorsäure ist.

8. Verfahren nach mindestens einem der Ansprüche 1 bis 7, wobei das mindestens eine chromatographische Verfahren SMB-Chromatographie, oder Chromatographie mit Ionentauschern ist.

9. Verfahren nach mindestens einem der Ansprüche 1 bis 8, wobei zwischen dem ersten und dem zweiten Verfahrensschritt ein Entsalzungsschritt zur Entfernung von Prozesschemikalien eingesetzt wird.

10. Verfahren nach Anspruch 9, wobei der Entsalzungsschritt mittels entsalzender Elektrodialyse erfolgt.

## Claims

1. A process for recovering an auxiliary agent employed in a fermentation process for preparing a product, which auxiliary agent was added to a fermentation broth, and from which another substance is formed in said fermentation broth by a chemical reaction, the following steps being passed through:
• the fermentation broth is subjected to a first process step in which said auxiliary agent employed in said fermentation process is an acidic ion exchanger, a mineral lye or mineral acid selected from the group consisting of aqueous sodium hydroxide solution, ammonia, aqueous potassium hydroxide solution, aqueous hydrochloric acid solution, sulfuric acid or phosphoric acid, and said other substance formed is selected from the group consisting of chlorides of sodium, potassium, ammonium; phosphates of sodium, potassium, ammonium; sulfates of sodium, potassium, ammonium and/or at least par-tially exhausted acidic ion exchangers, is separated from the at least one product obtained in the fermentation process by at least one chromatographic method in a fraction A, and said product is obtained in a fraction B;
• in a second process step, said fraction A is subjected to a bipolar electrodialysis to generate from another substance formed from said auxiliary agent contained in fraction A, such as salts, said auxiliary agent, such as acids/lyes corresponding to the salts.

2. The process according to claim 1, wherein components not dissolved in said fermentation broth are separated into a fraction C in a step upstream from said first process step.

3. The process according to claim 2, wherein at least one filtration or ultrafiltration is employed for separating components not dissolved in said fermentation broth.

4. The process according to at least one of claims 1 to 3, wherein cells, enzymes, insoluble starch, nutrients, such as peptone, peptides from meat extract or yeast cells or yeast hydrolysate or yeast extract are present in fraction C.

5. The process according to at least one of claims 1 to 4, wherein the auxiliary agents are recirculated to the fermentation, especially after a predetermined concentration has been adjusted.

6. The process according to at least one of claims 1 to 5, wherein said at least one product obtained in the fermentation process is lactic acid, succinic acid, itaconic acid, citric acid, and other organic acids.

7. The process according to at least one of claims 1 to 6, wherein said auxiliary agent is aqueous sodium hydroxide solution, ammonia, aqueous potassium hydroxide solution, aqueous hydrochloric acid solution, sulfuric acid, phosphoric acid.

8. The process according to at least one of claims 1 to 7, wherein said at least one chromatographic method is SMB chromatography or chromatography with ion exchangers.

9. The process according to at least one of claims 1 to 8, wherein a desalting step for removing process chemicals is employed between said first and second process steps.

10. The process according to claim 9, wherein said desalting step is performed by means of desalting electrodialysis.

## Revendications

1. Procédé destiné à la récupération d'un additif qui est employé dans un processus de fermentation pour la fabrication d'un produit, additif qui a été ajouté à un bouillon de fermentation, et à partir duquel une autre substance est obtenue au moyen d'une réaction chimique dans le bouillon de fermentation, les étapes suivantes étant réalisées .
• le bouillon de fermentation est soumis à une première étape du procédé, dans laquelle l'additif employé dans le processus de fermentation est un échangeur d'ions acide, une lessive minérale ou un acide minéral, sélectionné parmi le groupe composé de la lessive de soude, l'ammoniaque, la lessive de potasse, l'acide chlorhydrique, l'acide sulfurique, ou l'acide phosphorique, et l'autre substance obtenue est sélectionnée parmi le groupe composé du chlorure de sodium, du chlorure de potassium, du chlorure d'ammonium, du phosphate de sodium, du phosphate de potassium, du phosphate d'ammonium, du sulfate de sodium, du sulfate de potassium, du sulfate d'ammonium et/ou des échangeurs d'ions acides au moins partiellement épuisés, est séparée en une fraction A, au moyen d'au moins un procédé chromatographique, de l'au moins un produit obtenu dans le processus de fermentation et ledit produit est obtenu dans une fraction B,
• dans une deuxième étape du procédé, la fraction A est soumise à une électrodialyse bipolaire afin de générer à partir de l'autre substance telle que des sels, obtenue à partir de l'additif contenu dans la fraction A, tel que des acides et/ou des lessives correspondant aux sels.

2. Procédé selon la revendication 1, dans lequel, dans une étape située en amont de la première étape du procédé des composants non dissous dans le bouillon de fermentation sont séparés en une fraction C.

3. Procédé selon la revendication 2, dans lequel au moins une filtration ou une ultrafiltration est employée en vue de la séparation des composants non dissous dans le bouillon de fermentation.

4. Procédé selon au moins l'une des revendications 1 à 3, dans lequel des cellules, des enzymes, de l'amidon insoluble, des nutriments tels que de la peptone, des peptides obtenus à partir d'extraits de viande ou des cellules de levures ou un hydrolysat de levure ou un extrait de levure sont présents dans la fraction C.

5. Procédé selon au moins l'une des revendications 1 à 4, dans lequel les additifs sont introduits de nouveau dans la fermentation, en particulier après la détermination d'une concentration définie au préalable.

6. Procédé selon au moins l'une des revendications 1 à 5, dans lequel au moins l'un des produits apparaissant dans le processus de fermentation est un acide lactique, un acide succinique, un acide itaconique, un acide citrique et d'autres acides organiques.

7. Procédé selon au moins l'une des revendications 1 à 6, dans lequel l'additif est de la lessive de soude, de l'ammoniaque, de lessive de potasse, de l'acide chlorhydrique, de l'acide sulfurique, de l'acide phosphorique.

8. Procédé selon au moins l'une des revendications 1 à 7, dans lequel au moins l'un des procédés chromatographiques est une chromatographie de type SMB ou une chromatographie avec des échangeurs d'ions acides.

9. Procédé selon au moins l'une des revendications 1 à 8, dans lequel une étape d'élimination des sels, destinée à la suppression des produits chimiques de processus, est employée entre la première étape et la deuxième étape du procédé.

10. Procédé selon la revendication 9, dans lequel l'étape d'élimination des sels a lieu au moyen d'une électrodialyse de dessalage.
